Europäisches Patentamt

European Patent Office

Office européen des brevets

(11) Veröffentlichungsnummer: **0 382 228**

**A1**

## (12) EUROPÄISCHE PATENTANMELDUNG

(21) Anmeldenummer: **90102516.3**

(22) Anmeldetag: **08.02.90**

(51) Int. Cl.⁵: **A61B 17/22**

(30) Priorität: **10.02.89 DE 3904049**

(43) Veröffentlichungstag der Anmeldung:
**16.08.90 Patentblatt 90/33**

(84) Benannte Vertragsstaaten:
**AT BE CH DE ES FR GB GR IT LI LU NL SE**

(71) Anmelder: **STORZ MEDICAL AG**
**Unterseestrasse 47**
**CH-8280 Kreuzlingen(CH)**

(72) Erfinder: **Hagelauer, Ulrich, Dr.**
**Moosfeldstrasse 6**
**D-8598 Bottighofen(DE)**

(74) Vertreter: **Münich, Wilhelm, Dr. et al**
**Willibaldstrasse 36**
**D-8000 München 21(DE)**

(54) Verfahren und Vorrichtung zur Kontrolle und Justierung von Elektroden.

(57) Beschrieben wird ein Verfahren und eine Vorrichtung zur Kontrolle und Justierung der Position von Elektroden (E1, E2), die eine sog. Unterwasser-Funkenstrecke insbesondere für medizinische Anwendungen bilden, und von denen eine mittels wenigstens eines Elektrodenhalters an einem Grundteil (G) befestigt ist.

Erfindungsgemäß wird an die Elektroden wird eine Justiervorrichtung angelegt, die Aufnahmen für die Elektroden in deren Sollposition bezüglich Achsversatz, Achswinkelfehler und Elektrodenabstand aufweist. Durch eine Relativ-Bewegung der Aufnahmen in einer Richtung senkrecht zur Elektrodenachse werden der bzw. die Elektrodenhalter plastisch derart verformt, daß sich die Elektroden nach der Rückfederung des oder der Elektrodenhalter in ihrer Sollposition befinden.

Fig.2a

Fig.2b

EP 0 382 228 A1

## Verfahren und Vorrichtung zur Kontrolle und Justierung von Elektroden

Die Erfindung bezieht sich auf ein Verfahren bzw. eine Vorrichtung zur Kontrolle und Justierung der Position von Elektroden, die eine sog. Unterwasser-Funkenstrecke insbesondere für medizinische Anwendungen bilden, und von denen eine mittels wenigstens eines Elektrodenhalters an einem Grundteil befestigt ist.

Beispielsweise zur extrakorporalen Lithotripsie werden sogenannte Unterwasserfunkenstrecken eingesetzt, bei denen ein Hochspannungs-Durchbruch zwischen zwei Elektroden ein Fluid schlagartig verdampft, so daß sich eine Stoßwellen-Front in Form einer Kugelwelle ausbreitet. Diese Kugelwelle kann dann beispielsweise mittels eines ellipsoidalen Reflektors auf einen Fokuspunkt gebündelt werden.

Dabei ist in der Regel eine Elektrode fest an einer Grundplatte oder dgl. angebracht, während die dieser Elektrode gegenüberstehende Elektrode von einer Reihe von Stäben gehalten wird, die eine "Art Käfig" bilden und an der vorstehend angesprochenen Grundplatte angebracht sind

Die technische Ausgestaltung einer solchen Unterwasser-Funkenstrecke kann mittels nachführbarer Elektroden (Fa. Technomed) oder in Form eines auswechselbaren Verschleißteils (Fa. Dornier) realisiert sein. In beiden Fällen hängt die Intensität, Druckanstiegsteilheit und Ortsreproduzier-barkeit der Stoßwellenfront entscheiden von der Beschaffenheit und Geometrie der Elektrodenspitzen ab. Die relevanten geometrischen Parameter sind der Abstand, die Fluchtung und der Winkelversatz der Elektrodenspitzen.

Bei der Justierung der Elektrodenspitzen müssen sehr geringe Toleranzen eingehalten werden, um einen reproduzierbaren Funkensprung zu erzielen. Dies führt zu einem erhöhten Aufwand bei der Produktion. Weiterhin kann es durch die hohe mechanische Belastung - infolge der sehr großen Drucke beim Funkensprung von einigen 1000 bar - zu einer Verformung der Elektrodenhalterungen kommen. Ein weiterer Aspekt ist die zwangsläufig eintretende Abstandsänderung der Elektroden durch den Abbrand. All diese Faktoren führen zu einer Fehljustierung der Elektroden, mit der Folge von Fehlfunktionen.

Diese Fehlfunktionen können erhebliche Risiken nach sich ziehen, da Lithotriptoren auf der Basis von Funkenstrecken häufig im medizinischen Bereich eingesetzt werden. Die verringerte Effektivität von fehljustierten Elektroden bewirkt eine höhere Schußzahl, damit eine verlängerte Behandlungs- und Narkosedauer. Im umgünstigsten Fall kommt es zu einer Verlagerung des therapeutischen Fokus, d.h. zu einer Verlagerung der Zone maximalen Drucks weg vom Konkrement und damit in benachbarte Organregionen. Das Risiko von Organblutungen, wie sie infolge der extrakorporalen Lithotripsie ohnehin häufig auftreten, wird hierdurch unnötig gesteigert und führt zu einer erhöhten Schmerzwirkung und Belastung des Patienten. Prekär wirken sich Fehljustierungen dann aus, wenn Konkremente in unmittelbarer Nähe von Gefäßen liegen, wie dies z.B. bei der Behandlung von Uretersteinen der Fall sein kann. Hier können Gefäßperforationen mit der Folge akut lebensbedrohender Zustände auftreten.

Bei den heute im Einsatz befindlichen Systemen hat der Anwender keine Möglichkeit, die Geometrie der Funkenstrekke zu kontrollieren oder zu justieren. Ebensowenig besteht die Möglichkeit, die durch Abbrand entstehende Vergrößerung des Elektrodenabstands wieder neu zu justieren. Damit werden - um die vorstehend erwähnten Fehlfunktionen wenigstens z.T. zu vermeiden - häufig eigentlich noch voll funktionsfähige Elektroden "routinemäßig ausgetauscht, was erhöhte Kosten pro Behandlung zur Folge hat.

Der Erfindung liegt die Aufgabe zugrunde, ein Verfahren zur Kontrolle und Justierung der Position von Elektroden, die eine sog. Unterwasser-Funkenstrecke insbesondere für medizinische Anwendungen bilden, und von denen eine mittels wenigstens eines Elektrodenhalters an einem Grundteil befestigt ist, sowie eine Vorrichtung zur Verwendung in dem Verfahren anzugeben, die eine Kontrolle und Justierung der geometrischen Parameter von Unterwasser-Funkenstrecken erlauben.

Eine erfindungsgemäße Lösung dieser Aufgabe ist mit ihren Weiterbildungen in den Patentansprüchen gekennzeichnet.

Erfindungsgemäß wird an die Elektroden eine Justiervorrichtung angelegt, die Aufnahmen für die Elektroden in deren Sollposition bezüglich Achsversatz, Achswinkelfehler und Elektrodenabstand aufweist. Durch eine Relativ-Bewegung der Aufnahmen in einer Richtung senkrecht zur Elektrodenachse werden der bzw. die Elektrodenhalter plastisch derart verformt, daß sich die Elektroden nach der Rückfederung des oder der Elektrodenhalter in ihrer Sollposition befinden.

Hierdurch kann auch ohne großen meßtechnischen Aufwand selbst ein vergleichsweise ungeübter Anwender die Geometrieparameter der Unterwasser-Funkenstrecke überprüfen und gegebenenfalls nachjustieren.

Das erfindungsgemäße Verfahren kann in gleichem Maße auch bei der Herstellung der Unterwasser-Funkenstrecke für die "Erst-Justierung" Verwendung finden.

Weiterbildungen der Erfindung sind in den Unteransprüchen gekennzeichnet.

Gemäß Anspruch 2 erfolgt die mechanische Justierung durch eine achssymmetrische Biegung des bzw. der Elektrodenhalter. Hierdurch ist gewährleistet, daß in einem Arbeitsgang sowohl Achsversatz als auch Winkelversatz ohne Veränderung des Elektrodenabstandes korrigiert werden können.

Im Anspruch 3 ist gekennzeichnet, daß der Elektroden-Abstand unabhängig von Achsversatz und Winkelfehler nachträglich veränderbar ist. Dies kann beispielsweise dadurch erfolgen, daß der Abstand der Aufnahmen zur Justierung des Elektrodenabstandes einstellbar ist (Anspruch 7).

In den Ansprüchen 4 folgende ist eine Justiervorrichtung insbesondere zur Durchführung des erfindungsgemäßen Verfahrens angegeben.

Die erfindungsgemäße Vorrichtung weist zwei teilbare Aufnahmen für die Elektroden auf, deren Innenkontur der Außenkontur der Elektroden angepaßt ist, und die sich in der Grundstellung der Vorrichtung in der Sollposition der Elektroden bezüglich Achsversatz, Achswinkelfehler und Elektrodenabstand befinden. Zur Durchführung des Justiervorgangs sind die Aufnahmen in einer Richtung senkrecht zur Elektrodenachse relativ zueinander bewegbar.

Bei der im Anspruch 5 angegebenen bevorzugten Weiterbildung der erfindungsgemäßen Vorrichtung ist eine der Aufnahmen feststehend, d.h. lediglich eine der Aufnahmen kann bewegt werden. In die feststehende Aufnahme wird die direkt an der Grundplatte angebrachte Elektrode eingesetzt, während die von den "Käfigstangen" gehaltene Elektrode in die bewegliche Aufnahme eingesetzt wird. Hierdurch können die Käfigstangen plastisch derart verformt werden, daß eine exakte Justierung erreicht wird.

In jedem Falle ist es bevorzugt, wenn die Bewegung eine Drehung um die Elektrodenachse ist (Anspruch 6), da hierdurch eine gleichmäßige Verformung sämtlicher Käfigstangen sichergestellt ist.

Durch die im Anspruch 8 angegebene Ausgestaltung, gemäß der die Vorrichtung zusätzliche Aufnahmen für die stabförmigen Elektrodenhalter aufweist, ist sichergestellt, daß die Käfig-Stangen lediglich in einem bestimmten Bereich verformt werden, nämlich in dem Bereich, der nicht von den Aufnahmen des "Biegewerkzeug" aufgenommen wird.

Die erfindungsgemäße Justiervorrichtung ist selbstverständlich nicht nur zum Justieren der Elektroden, sondern auch nur zur Überprüfung deren Position verwendbar; hierzu ist gemäß Anspruch 9 ein Teil der Vorrichtung als Lehre zur Kontrolle der Elektrodenposition verwendbar.

Dabei kann die Überprüfung u.a. dadurch erfolgen, daß gemäß Anspruch 10 ein optisches System vorgesehen ist, das die Elektrodenspitzen zur Kontrolle unter Sicht oder mittels einer Bildverarbeitungseinrichtung abbildet. Insbesondere bei Verwendung eines Bildverarbeitungssystems kann die Kontrolle und Justierung der Elektroden-Position mittels eines automatischen Systems erfolgen. Dabei kann beispielsweise ein Servomotor die Bewegung der einzelnen Vorrichtungsteile gegeinander steuern.

Die Erfassung der Elektroden-Position kann gemäß Anspruch 11 aber auch dadurch (zusätzlich) erfolgen, daß die Aufnahmen Kontaktelemente für die Elektrodenspitzen aufweisen, die in der Sollstellung der Elektroden mit diesen elektrisch leitend verbunden sind. Das Signal der Kontaktelemente kann gemäß Anspruch 12 an eine Steuereinheit angelegt sein, die beispielsweise einen Mikropozessor aufweist, und die die Relativbewegung der Aufnahmen entsprechend dem Signal der Kontaktelement steuert.

Die Erfindung wird nachstehend anhand eines Ausführungsbeispiels unter Bezugnahme auf die Zeichnung näher beschrieben, in der zeigen:

Fig. 1 die bei der Justierung von eine Unterwasser-Funkenstrecke bildenden Elektroden relevanten Geometrie-Parameter,

Fig. 2a und b die Aufhängung der Elektroden, und

Fig. 3 eine erfindungsgemäße Justiervorrichtung.

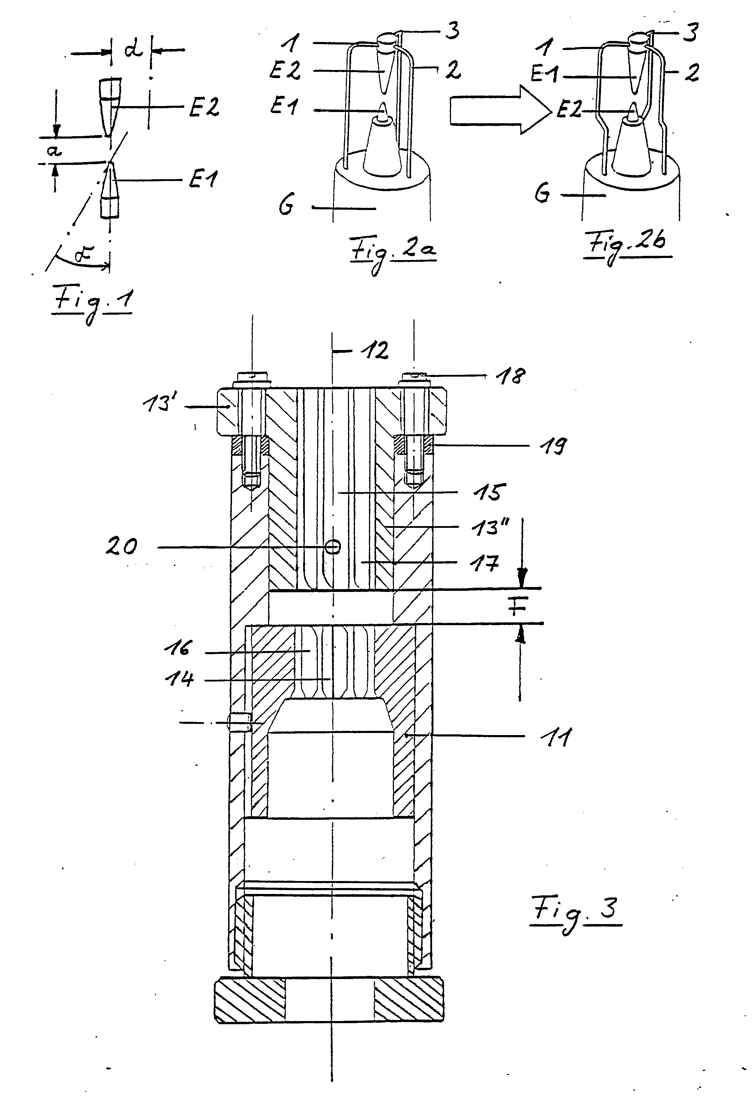

Fig. 1 zeigt die bei der Justierung von eine UnterwasserFunkenstrecke bildenden Elektroden E1 und E2 relevanten Geometrie-Parameter, nämlich den Winkelfehler $\alpha$, den Abstand a der beiden Elektroden E1 und E2 sowie den Achsversatz d der Elektroden.

Fig. 2a zeigt die "Aufhängung" der einander gegenüberstehenden Elektroden E1 und E2. Die Elektrode E1 ist an einem Grundteil bzw. einer Grundplatt G angebracht. Die Elektrode E2 wird von Stangen 1 bis 3 gehalten, die eine "Art Käfig" bilden, und die Ausbreitung der erzeugten kugelförmigen Stoßwelle praktisch nicht behindern. Die Stangen 1 bis 3 wiederum sind an dem Grundteil G angebracht.

Wie bereits ausgeführt, kann durch eine Vielzahl von Gründen - beispielsweise Herstellfehler, die hohe mechanische Belastung beim Funkensprung oder Abbrand - eine Abweichung der Position der Elektrodenspitzen von ihrer Soll-Positon entstehen.

Erfindungsgemäß erfolgt die Justierung der Elektroden dadurch, daß durch eine Relativ-Bewegung der Aufnahmen in einer Richtung senkrecht zur Elektrodenachse die Stangen 1, 2 und 3 plastisch derart verformt werden, daß sich die Elektroden nach der Rückfederung des oder der Elektrodenhalter in ihrer Sollposition befinden.

Hierzu wird die in Fig. 3 dargestellte Justiervorrichtung verwendet:

Die Vorrichtung weist ein feststehendes Biegewerkzeug 11 und ein gegen das feststehende Biegewerkzeug 11 um eine Achse 12 drehbares Biegewerkzeug 13 auf. Die Biegewerkzeuge weisen Aufnahmen 14 bzw. 15 für die Elektroden E1 bzw. E2 und Aufnahmen für die Käfig-Stäbe 1 bis 3 auf, von denen in Fig. 3 nur die Aufnahmen 16 und 17 dargestellt sind. Weiterhin ist die Justiervorrichtung teilbar, so daß sie an die Elektroden E1 und E2 sowie die Stäbe 1 bis 3 angelegt und von diesem wieder abnehmbar ist.

Ferner besteht das drehbare Biegewerkzeug 13 aus zwei Teilen 13′ und 13″, die miteinander mittels Schrauben 18 verschraubt sind. Somit ist es durch Einlegen von Distanzscheiben 19 möglich, den freien Abstand F zwischen den Aufnahmen einzustellen. Der Grund hierfür wird weiter unten näher erläutert werden.

Weiterhin weist die Justiervorrichtung mindestens ein Fenster 20 auf, das eine Kontrolle des Justierergebnisses gestattet.

Im folgenden soll die Funktionsweise der erfindungsgemäßen Vorrichtung beschrieben werden:

Das Justier-Prinzip beruht auf einer plastischen Verformung der Elektrodenhalter 1 bis 3 in Form einer axialsymmetrischen Biegung des oberen Teils gegen den unteren Teil der Elektrodenhalter (Fig. 2b). Unabhängig von einer bereits vorliegenden unsymmetrischen Verformung - sie bewirkt lediglich einen früheren oder späteren Fließbeginn in den einzelnen Stangen 1 bis 3 -weist die Elektrodenhalterung nach der Verformung die erforderliche Form auf, um Flucht- und Achsversatzfehler innerhalb enger Grenzen zu halten. Durch entsprechende Dimensionierungen des Freiraumes F wird erreicht, daß die elastische Rückfederung genau dem einzustellenden Abstandsmaß a entspricht.

Ein weiterer Vorteil der erfindungsgemäßen Vorrichtung besteht darin, daß in einem Arbeitsgang alle drei genannten geometrischen Parameter justierbar sind.

Daraus ergibt sich eine universelle Verwendbarkeit der Vorrichtung

a) als Justiervorrichtung in der Fertigung von Unterwasser-Funkenstrecken, besonders zur Behebung von Flucht- und Achsversatz-Fehlern, und

b) zum Nachjustieren des Elektroden-Abstands.

Die Justierung kann unter optischer Sicht oder automatisch anhand der erfaßten Maßabweichung erfolgen.

Die Maßabweichung kann dabei mittels einer optischen Meßeinrichtung erfaßt werden, die die Lage der Elektrodenspitzen zueinander bestimmt. Die Kontrolle kann visuell, beispielsweise mittels eines Meßmikroskops oder über eine Kamera mit nachgeschalteter Bildverarbeitungseinrichtung erfolgen. Die Biegevorrichtung weist hierzu die optischen Fenster 20 auf, um mit Hilfe des Meßmikroskops oder von elektronischer Bildverarbeitung die Elektrodenposition vor und nach der Justierung kontrollieren zu können. Eine Kontrolle der Maßabweichung ist ferner durch geeignete Kontaktelement in den Aufnahmen für die Elektroden möglich.

## Ansprüche

1. Verfahren zur Kontrolle und Justierung der Position von Elektroden, die eine sog. Unterwasser-Funkenstrecke insbesondere für medizinische Anwendungen bilden, und von denen eine mittels wenigstens eines Elektrodenhalters an einem Grundteil befestigt ist,

**gekennzeichnet** durch folgende Verfahrensschritte:
- an die Elektroden wird eine Justiervorrichtung angelegt, die Aufnahmen für die Elektroden in deren Sollposition bezüglich Achsversatz, Achswinkelfehler und Elektrodenabstand aufweist,
- durch eine Relativ-Bewegung der Aufnahmen in einer Richtung senkrecht zur Elektrodenachse werden der bzw. die Elektrodenhalter plastisch derart verformt, daß sich die Elektroden nach der Rückfederung des oder der Elektrodenhalter in ihrer Sollposition befinden.

2. Verfahren nach Anspruch 1,
dadurch **gekennzeichnet**, daß die mechanische Justierung durch eine achssymmetrische Biegung des bzw. der Elektrodenhalter erfolgt.

3. Verfahren nach Anspruch 1 oder 2,
dadurch **gekennzeichnet**, daß der Elektroden-Abstand unabhängig von Achsversatz und Winkelfehler nachträglich veränderbar ist.

4. Justiervorrichtung insbesondere zur Durchführung des Verfahrens nach einem der Ansprüche 1 bis 3,
dadurch **gekennzeichnet**, daß zwei teilbare Aufnahmen für die Elektroden vorgesehen sind, deren Innenkontur der Außenkontur der Elektroden angepaßt ist, und die sich in der Grundstellung der Vorrichtung in der Sollposition der Elektroden bezüglich Achsversatz, Achswinkelfehler und Elektrodenabstand befinden, und
daß die Aufnahmen in einer Richtung senkrecht zur Elektrodenachse relativ zueinander bewegbar sind.

5. Vorrichtung nach Anspruch 4,
dadurch **gekennzeichnet**, daß eine Aufnahme feststehend ist.

6. Vorrichtung nach Anspruch 4 oder 5,
dadurch **gekennzeichnet**, daß die Bewegung eine Drehung um die Elektrodenachse ist.

7. Vorrichtung nach einem der Ansprüche 4 bis 6,

dadurch **gekennzeichnet**, daß der Abstand der Aufnahmen zur Justierung des Elektrodenabstandes einstellbar ist.

8. Vorrichtung nach einem der Ansprüche 4 bis 7, dadurch **gekennzeichnet**, daß die Vorrichtung zusätzliche Aufnahmen für die stabförmigen Elektrodenhalter aufweist.

9. Vorrichtung nach einem der Ansprüche 4 bis 8, dadurch **gekennzeichnet**, daß ein Teil der Vorrichtung als Lehre zur Kontrolle der Elektrodenposition verwendbar ist.

10. Vorrichtung nach einem der Ansprüche 4 bis 9, dadurch **gekennzeichnet**, daß ein optisches System vorgesehen ist, das die Elektrodenspitzen zur Kontrolle unter Sicht oder mittels einer Bildverarbeitungseinrichtung abbildet.

11. Vorrichtung nach einem der Ansprüche 4 bis 10, dadurch **gekennzeichnet**, daß die Aufnahmen Kontaktelemente für die Elektrodenspitzen aufweisen, die in der Sollstellung der Elektroden mit diesen elektrisch leitend verbunden sind.

12. Vorrichtung nach Anspruch 10 oder 11, dadurch **gekennzeichnet**, daß eine Steuereinheit die Relativbewegung der Aufnahmen entsprechend dem Signal der Kontaktelemente oder der Bildverarbeitungseinrichtung steuert.

g0102516

Fig.1

Fig.2a

Fig.2b

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 124 686 (DORNIER) <br> * Seite 9, Zeilen 12-16; Figur 3 * <br> ----- | 1,4,6 | A 61 B 17/22 |

**RECHERCHIERTE SACHGEBIETE (Int. Cl.5)**

A 61 B
H 01 T
G 10 K

Der vorliegende Recherchenbericht wurde für alle Patentansprüche erstellt

| Recherchenort | Abschlußdatum der Recherche | Prüfer |
|---|---|---|
| DEN HAAG | 09-05-1990 | MOERS R.J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument

........................................................................
& : Mitglied der gleichen Patentfamilie, übereinstimmendes
    Dokument